# EUROPEAN PATENT SPECIFICATION

(11) **EP 3 714 662 B1**
(45) Date of publication and mention of the grant of the patent: **24.01.2024**
(21) Application number: 18803444.1
(22) Date of filing: 21.11.2018
(51) Int. Cl.: H05B 47/115, A61N 5/06, H05B 47/125, H05B 47/19

(54) **A METHOD FOR GENERATING A DATABASE**
VERFAHREN ZUM ERZEUGEN EINER DATENBANK
PROCÉDÉ DE GÉNÉRATION D'UNE BASE DE DONNÉES

(30) Priority: 23.11.2017 EP 17203325
(43) Date of publication of application: 30.09.2020
(73) Proprietor: BrainLit AB, 223 63 Lund (SE)
(72) Inventor: WINGREN, Tord, 222 36 Lund (SE); LÖWGREN, Truls, 216 19 Malmö (SE)
(74) Representative: Zacco Sweden AB
(86) International application number: PCT/EP2018/082030
(87) International publication number: WO 2019/101778

(56) References cited:
- WO-A1-2013/141792
- WO-A1-2016/019005
- WO-A1-2017/151682
- US-A1- 2008 146 892

## Description

### Technical field

The present invention relates to a method for generating a database to be used to set illumination of an adjustable light source according to claim 1 and to a light control system according to claim 8. Preferred embodiments of the invention are defined in the dependent claims.

### Background

It has become known that many humans or animals have different lighting needs, that is they are affected by the light in their environment in different ways and therefore have different needs when it comes to the surrounding light. Some of these needs stem from physical conditions, for example, eye problems, some stem from mental conditions, for example, winter depressions and some stem from individual preference.

WO 2016/019005 provides methods and systems for setting up light scenes. These methods and system automate the control of variable parameters after defining a set of fixtures and static properties of the fixtures in a given environment and a selected mood.

WO 2017/151682 discloses a wireless control system that includes multiple wireless transmitters and a control circuit configured to transmit information from the wireless transmitters, wherein the control circuit is configured to selectably enable and disable each of the wireless transmitters.

To this end, the health condition and/or behavior of a human and/or an animal is influenced by the light it is exposed to, i.e. light intensity and/or spectral content of the light. As an example, the blue component of the light in the morning stimulating alertness. In the evening, the natural light turns redder which improves relaxation. More specifically, it is known that the blue light in the morning increases cortisol and reduces melatonin increasing attentiveness and focus, while the warmer red tone increases the body's melatonin and reduces cortisol causing the relaxation.

There is therefore is a need for efficient methods and systems for providing light exposure which is adapted to the needs of individuals.

### Summary of the invention

In view of the above, it is an object of the present invention to provide a method for generating a database to be used to set illumination of an adjustable light source. It is further an object to mitigate, alleviate or eliminate one or more of the above-identified deficiencies in the art and disadvantages singly or in any combination and solve at least the above mentioned problem.

According to a first aspect: a method for generating a database to be used to set illumination of an adjustable light source is provided. The method comprising: for a plurality of different users: identifying an individual user; setting a specific light profile of the adjustable light source, the specific light profile comprising information pertaining to a spectral content and/or intensity of light by which the individual user is exposed to; while exposing the individual user for the specific light profile, sensing data pertaining to a health condition and/or a behavior of the individual user, wherein the act of sensing comprises sensing one or more of: pulse, heart rate variability, blood pressure, sleep pattern, eating habits, facial expression, oxygen level, body posture, tone of voice, hormone levels, movement pattern, and productivity; determining a data set linking the specific light profile, genomic data pertaining to the individual user, and the data pertaining to the health condition and/or the behavior of the individual user sensed while exposing the individual user to the specific light profile; and storing the linked data set in a database.

The database links light exposure from the adjustable light source, i.e. the spectral content and/or intensity of light exposure, of an individual user with the health condition and/or the behavior of the individual user as well as the genomic data of the individual user. The database is therefore obtained which allows for matching of a specific light exposure, genomic data and a health condition and/or a behavior of an individual user.

As an example, the linked data, e.g. stored as entries in the database allows for correlation of a given health condition, a specific light exposure to a specific genomic data. The database thereby provides light recipes, i.e. directives, for achieving a given health condition and/or a behavior of an individual user having a given genomic data. The database may therefore be used to set a suitable light exposure for a new user provided that the genomic data of the new user matches or at least partly matches genomic data stored of the database. As a result, a biocentric light exposure may be obtained in that the light exposure takes account of the individual genomic data and preference of the individual user. A better specificity in the light exposure may thereby be obtained. An improved starting point for achieving a desirable health condition and/or behavior of an individual user is further provided by the database providing information pertaining to the light exposure to be used to induce the desirable health condition and/or behavior.

The wording "genomic data" may be construed as data pertaining to the genome or at least a portion of the genome of an individual user. The genome may be understood as the genetic material of an organism such as a human, an animal or a plant. The genome comprises DNA. The genome is the total genetic material of an organism and includes both the genes and non-coding sequences. The genome further comprises genetic material of the mitochondria and chloroplasts. To this end, genomics aims at the collective characterization and quantification of genes, which direct the production of proteins with the assistance of enzymes and messenger molecules. Genomics further involves the sequencing and analysis of genomes.

The genomic data may comprise the genome of an individual.

The genomic data may comprise at least a portion of the genome of an individual user.

The wording "health condition" may relate to both physical health and mental health. An example of a mental health condition that is influenced by light is winter/darkness depressions which are often treated with light therapy. An example of physical health condition is eye problems which may render a user's eyes very sensitive to certain amounts of light.

The wording "behavior of an individual user" may be understood as the response of an individual such as a human or an animal to internal and external stimuli. The wording "behavior of an individual user" may be understood as the physical state and/or motion associated with the individual user. The "behavior of an individual user" may be an observable emotional state. The observable emotion may, for example, be a facial expression, a body posture or a movement pattern.

The wording "light profile" may be construed as the spectral content and/or intensity of light. The spectral content may further be construed as a measure of the amount of electromagnetic radiation emitted at different wavelengths. In other words, the spectral content may be referred to as the spectrum of the light. The spectral content of light or its spectrum may be determined by a spectrograph dispersing the light from a light source into its component wavelengths so that it can be recorded then analyzed.

The determining of the data set linking the specific light profile, genomic data pertaining to the individual user, and the data pertaining to the health condition and/or the behavior of the individual user sensed while exposing the individual user to the specific light profile may further comprise determining a point in time at which the individual user is exposed to the specific light profile.

An advantage being that the time the exposure took place may be determined. The point in time may further be correlated to the light exposure and stored in the database.

The determining of the data set linking the specific light profile, genomic data pertaining to the individual user, and the data pertaining to the health condition and/or the behavior of the individual user sensed while exposing the individual user to the specific light profile may further comprise determining a time of exposure during which the individual user is exposed to the specific light profile.

An advantage being that the dosage of the exposure may be determined. The time of exposure may further be stored in the database.

The method may further comprise, for a specific individual user:
exposing the specific individual user for different specific light profiles;
sensing data pertaining to a health condition and/or a behavior of the specific individual user for each different specific light profile; and
linking each different specific light profile, genomic data pertaining to the specific individual user, and the data pertaining to the health condition and/or the behavior of the specific individual user sensed while exposing the specific individual user for each different specific light profile.

The database may thereby be built up faster. A better correlation of the genomic data pertaining to an individual user to the different specific light exposures, and the associated health condition and/or a behavior of an individual user may further be obtained.

The wording "specific individual user" may be understood as a user selected among a plurality of different users.

Linking each different specific light profile, genomic data pertaining to the specific individual user, and the data pertaining to the health condition and/or the behavior of the specific individual user sensed while exposing the specific individual user for each different specific light profile may further comprise points in time at which the individual user is respectively exposed to each different specific light profile.

Linking each different specific light profile, genomic data pertaining to the specific individual user, and the data pertaining to the health condition and/or the behavior of the specific individual user sensed while exposing the specific individual user for each different specific light profile may further comprise times of exposure during which the individual user is respectively exposed to each different specific light profile.

A specific light profile may be selected from a set of predetermined specific light profiles. Light profiles known to influence the health condition and/or behavior of an individual may thereby be used.

Known light profiles may thereby be used for building up the database.

According to a second aspect a light control system is provided. The light control system comprising: an adjustable light source, adjustable in spectral content and/or intensity of light emitted; a first sensor configured to identify an individual user among a plurality of individual users; a central control engine configured to set a specific light profile of the adjustable light source, the specific light profile comprising information pertaining to a spectral content and/or intensity of light by which the individual user is exposed to; a second sensor configured to sense data pertaining to a health condition and/or a behavior of the individual user while exposing the individual user to the specific light profile, wherein the second sensor is configured to sense one or more of pulse, heart rate variability, blood pressure, sleep pattern, eating habits, facial expression, oxygen level, body posture, tone of voice, hormone levels, movement pattern, and productivity; a first database comprising genomic data pertaining to each of the plurality of individual users; and wherein the central control engine is further configured to: determine a data set linking the specific light profile, genomic data pertaining to the individual user, and the data pertaining to the health condition and/or the behavior of the individual user sensed by the second sensor; and store the linked data set in a second database.

The light control system allows for building up of a data base for providing customized light to individuals.

The first sensor and second sensor may be the same sensor.

The first sensor may be a camera.

The central control engine may further be configured to determine a point in time at which the individual user is exposed to the specific light profile.

The central control engine may further be configured to determine a time of exposure during which the individual user is exposed to the specific light profile.

The first database and the second database may be the same database.

The above mentioned features of the first aspect, when applicable, apply to this second aspect as well. In order to avoid undue repetition, reference is made to the above.

A further scope of applicability of the present invention will become apparent from the detailed description given below. However, it should be understood that the detailed description and specific examples, while indicating preferred embodiments of the invention, are given by way of illustration only, since various changes and modifications within the scope of the invention will become apparent to those skilled in the art from this detailed description.

Hence, it is to be understood that this invention is not limited to the particular component parts of the device described or steps of the methods described as such device and method may vary. It is also to be understood that the terminology used herein is for purpose of describing particular embodiments only, and is not intended to be limiting. It must be noted that, as used in the specification and the appended claim, the articles "a," "an," "the," and "said" are intended to mean that there are one or more of the elements unless the context clearly dictates otherwise. Thus, for example, reference to "a unit" or "the unit" may include several devices, and the like. Furthermore, the words "comprising", "including", "containing" and similar wordings do not exclude other elements or steps.

### Brief Description of the Drawings

The above and other aspects of the present invention will now be described in more detail, with reference to appended drawings showing embodiments of the invention. The figures should not be considered limiting the invention to the specific embodiment; instead they are used for explaining and understanding the invention.

As illustrated in the figures, the sizes of layers and regions are exaggerated for illustrative purposes and, thus, are provided to illustrate the general structures of embodiments of the present invention. Like reference numerals refer to like elements throughout.
Fig. 1 is a schematic view of a space and a light control system.
Fig. 2 illustrates components of the light control system of Fig. 1.
Fig. 3 is a block scheme of a method for generating a database to be used to set illumination of an adjustable light source.
Fig. 4a is a schematic view of a space and a light control system wherein an individual user is exposed to light having a specific light profile.
Fig. 4b is a schematic view of the space and the light control system of Fig. 4a, wherein the individual user is exposed to light having a specific light profile being different to the specific light profile of Fig. 4a.

### Detailed description

The present invention will now be described more fully hereinafter with reference to the accompanying drawings, in which currently preferred embodiments of the invention are shown. This invention may, however, be embodied in many different forms and should not be construed as limited to the embodiments set forth herein; rather, these embodiments are provided for thoroughness and completeness, and to fully convey the scope of the invention to the skilled person.

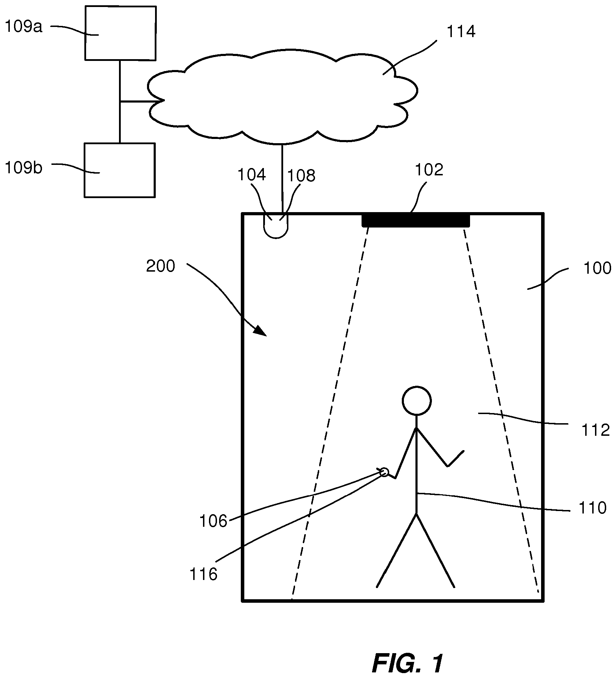

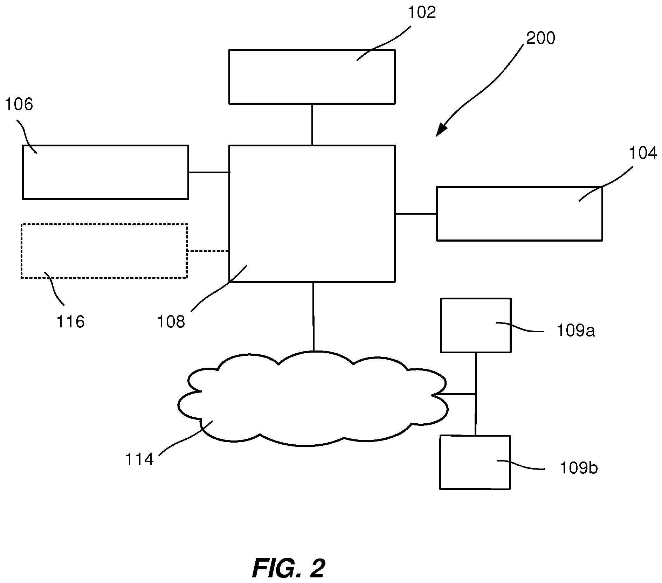

In the following a method for generating a database to be used to set illumination of an adjustable light source and a light control system are described in relation to Figs. 1 and 2.

Fig. 1 is a schematic view of a space 100 and a light control system 200, see also Fig. 2 illustrating components of the light control system 200.

The light control system 200 comprises an adjustable light source 102, a first sensor 104, a second sensor 106, a central control engine 108, a first database 109a and a second database 109b.

The adjustable light source 102, the first sensor 104, the second sensor 106, and the central control engine 108 may be configured to communicate via wired or wireless communication in a communication network.

The central control engine 108 is illustrated to be comprised in the first sensor 104, but may be arranged as a separate unit.

The central control engine 108 may be arranged within the adjustable light source 102.

In other words, the central control engine 108 may be physically separate from the first sensor 104.

The central control engine 108 may form or be implemented in a separate unit in the space 100.

The central control engine 108 may alternatively be implemented on a remote server or on a central server or as part of a cloud service.

The central control engine 108 and the first sensor 104 may communicate with each other over a wired or wireless communication network. The central control engine 108 may be comprised in a single device. Alternatively, the central control engine 108 may be distributed over a plurality of devices.

The adjustable light source 102 is adjustable in spectral content and/or intensity of light emitted. An individual user 110 may be exposed 112 to the light emitted by the adjustable light source 102.

The first sensor 104 is configured to identify the individual user 110 among a plurality of individual users.

The first sensor 104 may be a digital camera. The digital camera may be arranged to identify the individual user 110 by capturing a single or a plurality of images. Image recognition may further be used to identify the individual user 110. The camera and/or the central control engine may be configured to perform the identification on an image or images captured by the digital camera. The image or images may be stored in a memory of the first sensor and/or of the central control engine.

The central control engine 108 is configured to set a specific light profile of the adjustable light source 102. The specific light profile comprises information pertaining to a spectral content and/or intensity of light by which the individual user 110 is exposed 112.

The second sensor 106 configured to sense data pertaining to a health condition and/or a behavior of the individual user 110 while the individual user 110 is exposed 112 to the specific light profile.

The second sensor 106 may be a wearable electronic device carried by the individual use. The second sensor 106 may be a wearable electronic device that can be worn on the body of the individual user 110. The second sensor 106 may be an implant or an accessory.

The second sensor may be configured to monitor the user's health condition and/or behavior. The second sensor may be in contact with the user to collect data about the user. The second sensor may measure the activity of the individual user. The second sensor may be referred to as an activity tracker. The activity tracker may monitor and track movement-related metrics such as distance covered by walking or running, calorie consumption. The second sensor may be configured to measure blood pressure. The second sensor may comprise a gyroscope and/or an accelerometer. The gyroscope and/or accelerometer, may be used to specify the movement of the individual user if the second sensor is worn by the individual user. The movement pattern determined by the second sensor may provide information about the health status and/or the behavior of the individual user. A gyroscope and/or an accelerometer may e.g. provide information about if the individual user is active or still. The gyroscope and/or an accelerometer may e.g. or if the user uses slow or fast movements providing information about the mood of the individual user.

The second sensor may communicate with the central control engine via wireless communication e.g. by Bluetooth, Zigbee, Wifi, RFID, or NFC.

The second sensor may e.g. be a mobile phone, a tablet, a keyfob, a smart watch, or a smart bracelet.

The first database 109a comprises genomic data pertaining to the each of the plurality of individual users.

The central control engine 108 is further configured to determine a data set linking the specific light profile, genomic data pertaining to the individual user 110, and the data pertaining to the health condition and/or the behavior of the individual user 110 sensed by the second sensor 106. The central control engine 108 in also configured to store the linked data set in a second database 109b.

Thus, the light control system 200 allows for building up of a data base for providing customized light to individuals.

The first database 109a and/or the second database 109b may be software and/or hardware implemented. The databases 109a and/or 109b may be located in one specific device. Alternatively, the databases 109a and/or 109a may be distributed among a plurality of devices. The databases 109a and/or 109a may be accessible via a computer network such the internet. The databases 109a and/or 109a may be a part of a cloud 114.

The first database 109a and the second database 109b may be the same database.

The first sensor 104 may be configured to sense data pertaining to a health condition and/or a behavior of the individual user while the individual user is exposed to the specific light profile. The first sensor may for example be a digital camera. The digital camera may be used to capture an image or images of the individual user.

Image recognition may further be used to determine the health status and/or the behavior of the individual user. Image recognition may for example determine if the individual user is happy or depressed. The first sensor and/or the central control engine may be configured to perform the identification on an image or images captured by the digital camera.

The first sensor may communicate with the central control engine via wireless communication e.g. by Bluetooth, Zigbee, Wifi, RFID, or NFC.

Thus, the first sensor and/or the second sensor may be configured to sense data pertaining to a health condition and/or a behavior of the individual user. One or more sensors may therefore be used for the sensing. A combination of data obtained by more than one sensors may further be used.

The first sensor may be physically separate from the second sensor.

The second sensor may form or be implemented in a separate unit in the space.

The first sensor and the second sensor may communicate with each other over a wired or wireless communication network.

The light control system 200 may comprise one or more light sensors, see Figs. 1 and 2. In the example of Fig. 1, the control system comprises one light sensor 116 worn by the user 110 of the light control system 200. The second sensor 106 may comprise the light sensor 116. The second sensor 106 may form the light sensor 116.

The light sensor 116 is arranged inside the space 100.

It is, however, realized that light sensors may also be arranged outside the space 100. According to other embodiments the light sensor(s) may be arranged on a ceiling of the space 100. The light sensor(s) may be arranged on a wall of the space 100. The light sensor(s) may be arranged on a floor of the space 100. Further, in case of the light control system comprising a plurality of light sensors one or more of the light sensors may be worn by users of the light control system and one or more of the light sensors may be arranged inside (or possibly also outside) the space 100.

The first sensor 104 may comprise the light sensor. The first sensor 104 may form the light sensor 116.

The light sensor(s) is arranged to measure illumination data in the space 100. The illumination data may comprise parameters pertaining to a spatial distribution of the spectral content of the light in the space 100 and a spatial distribution of the light intensity in the space 100. The illumination data represents the light exposure 112 of the individual user 110 in the space 100. The light exposure 112 pertains to the light emitted by the adjustable light source 102. The light sensor 116 may further measure ambient light present in the space. The ambient light may, for example, originate from light entering the space from a window. The ambient light may be natural light originating from sun light.

The light sensors, e.g. the light sensor 116, are further configured to communicate the measurement to the central control engine 120. As a nonlimiting example, a light sensor may comprise an image sensor. Other nonlimiting examples of light sensors are photo sensors, biosensors, gas ionic sensors. The image sensor may be comprised in a camera.

The camera may be a digital camera.

The adjustable light source 102 may have an integrated light sensor. The integrated light sensor may be configured to sense properties of the actual transmitted light from the adjustable light source 102.

The light sensor may communicate with the central control engine via wireless communication e.g. by Bluetooth, Zigbee, Wifi, RFID, or NFC.

The central control engine 108 may further be configured to determine a data set linking the specific light profile, genomic data pertaining to the individual user 110, and the data pertaining to the health condition and/or the behavior of the individual user 110 sensed by the second sensor 106, and the illumination data comprising parameters pertaining to a spatial distribution of the spectral content of the light in the space 100 and a spatial distribution of the light intensity in the space 100.As an example, the linked data, e.g. stored as entries in the database 109b allows for correlation of a given health condition, the light exposure of the individual user in the space 100 to a specific genomic data. The light exposure of the individual user 110 pertains to the light emitted by the adjustable light source 102 and the spatial distribution of the spectral content of the light in the space 100 and a spatial distribution of the light intensity in the space 100. A better determination of the actual light content that the individual user is exposed to may thereby be achieved.

The light control system may further comprise a plurality of adjustable light sources.

The adjustable light source may be solid state based light source, e.g. light based on LED-, OLED-, AMOLED-, Electro Luminescent wire-, or LASER-technology. The adjustable light source may form part of a light panel comprising an adjustable light source or a plurality of adjustable light source.

The light panel may, for example, comprise different light sources, each emitting light with a certain spectral range such that an adjustable light source is achieved. One or several of the light sources within the light panel may emit visible light, ultra-violet light or infra-red light.

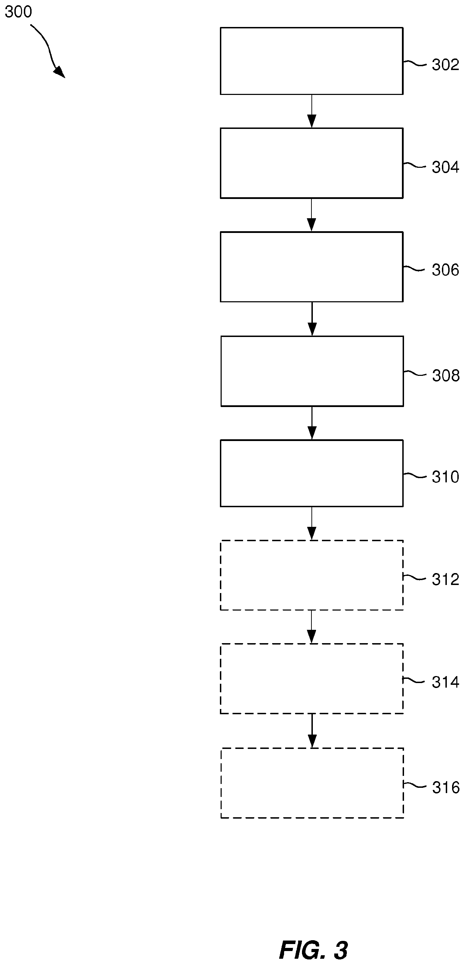

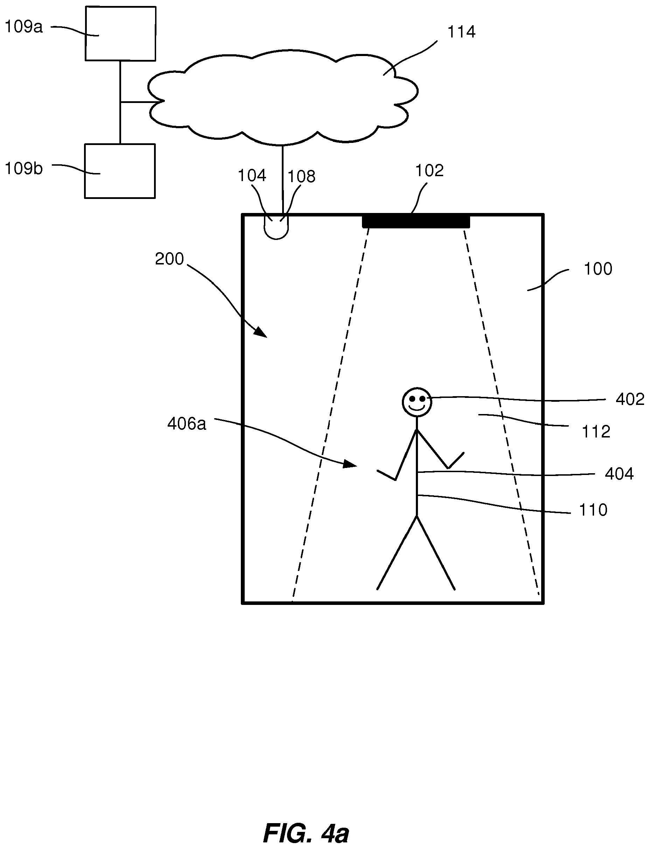

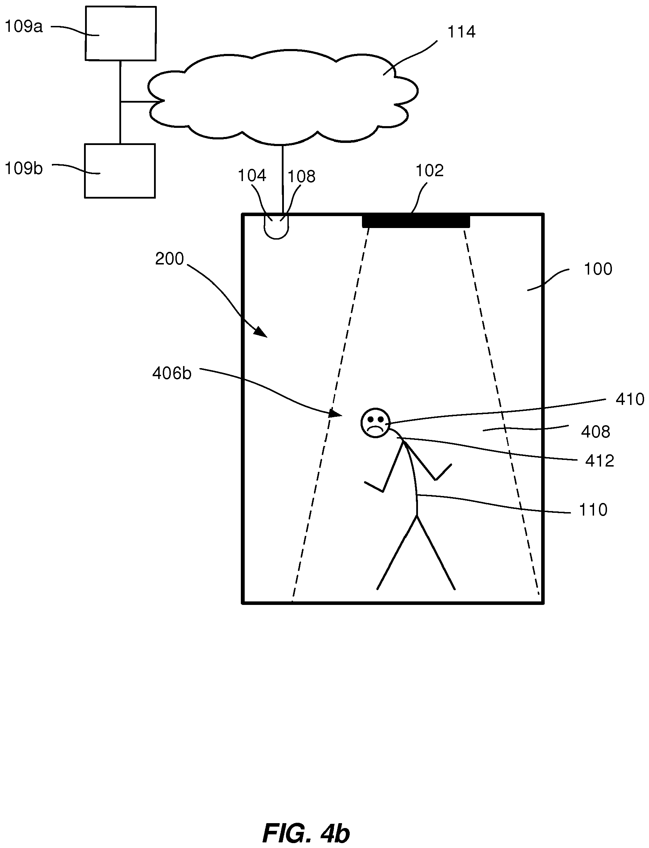

Next, an example of the method for generating a database to be used to set illumination of an adjustable light source is described with reference to Figs. 3, 4a and 4b.

The method 300 comprises, for a plurality of different users, identifying 302 an individual user 110. The identifying 302 may be performed by the first sensor described above, e.g. by face recognition.

The method 300 further comprises setting 304 a specific light profile of the adjustable light source 102. The specific light profile comprises information pertaining to a spectral content and/or intensity of light by which the individual user 110 is exposed 112.

The method 300 further comprises, while exposing 112 the individual user 110 for the specific light profile, sensing data 306 pertaining to a health condition and/or a behavior of the individual user, determining 308 a data set linking the specific light profile, genomic data, e.g. retrieved from the first database 109a, pertaining to the individual user 110, and the data pertaining to the health condition and/or the behavior of the individual user 110 as sensed 306 while exposing 112 the individual user 110 to the specific light profile, and storing 310 the linked data set in a database, e.g. the second database 109b as discussed above.

In figure 4a, the sensing data 306 is exemplified by the first sensor 104, being a digital camera, determining a health condition and/or behavior of the individual user 106. The camera may, for example, determine that the individual user 106 has a happy facial expression 402 and/or an upright body posture 404 indicating a health condition and/or behavior of the individual user, which may be characterized as happy and strong.

The entry in the database formed by the method 300 thereby links the light exposure 112 from the adjustable light source 102 with the health condition and/or the behavior of the individual user 110 as well as the genomic data of the individual user 110. The database 109b is therefore built up which allows for matching of a specific light exposure, genomic data and a health condition and/or a behavior of an individual user 110.

The above example illustrates that a happy and strong health condition and/or behavior of the individual user may be linked to the specific light exposure 112 pertaining to the specific light profile, of an individual user having a specific genomic data.

The method 300 may further comprise, for a specific individual user 106: exposing 312 the specific individual user 110 for different specific light profiles 406a and 406b, illustrated by the light exposure 112 and 408 in Figs. 4a and 4b, respectively.

The method 300 further comprising sensing data 314 pertaining to a health condition and/or a behavior of the specific individual user 110 for each different specific light profile. The method 300 then links 316 each different specific light profile, genomic data pertaining to the specific individual user 110, and the data pertaining to the health condition and/or the behavior of the specific individual user sensed 314 while exposing 112, 114 the specific individual user 110 for each different specific light profile.

The database may thereby be built up faster. A better correlation of the genomic data pertaining to an individual user to the different specific light exposures, and the associated health condition and/or a behavior of an individual user may further be obtained.

The different specific light profiles 406a and 406b, illustrated by the light exposure 112 and 408 in Figs. 4a and 4b, respectively may be different in spectral content and/or light intensity.

In figure 4b, the sensing data 314 is exemplified by the first sensor 104, again being a digital camera, determining a health condition and/or behavior of the individual user 110. The camera may, for example, determine that the individual user 110 has a sad facial expression 410 and a forward bent body posture 412 indicating a health condition and/or behavior of the individual user which may be described as sad and distressed.

Thus a sad and distressed health condition and/or behavior of the individual user may be linked to the light exposure 408 pertaining to a different specific light profile than the light exposure 112 above for the individual user having a given genomic data.

By way of example, the light exposure 112 may have a first spectral light distribution having a first main light intensity peak about a first peak wavelength providing light stimulation. The light exposure 408 may have a second light source configured to emit light having a second spectral light distribution having a second main light intensity peak about a second peak wavelength being different from the first peak wavelength.

The wording light stimulation may be construed as light suitable for inducing a biological and/or behavioral effect on a human or animal. The biological and/or behavioral effects may include a change in melatonin secretion, body temperature, cortisol secretion, heart rate, alertness, cognitive performance, psychomotor performance, brain blood flow and/or EEG response.

The first peak wavelength may be within the range of 450 - 512 nm, preferably 450 - 490 nm.

The adjustable light source may be a cold white light emitter and the first peak wavelength is within the range of 450 - 490 nm.

The adjustable light source may be a cold white light emitter and the first peak wavelength is within the range of 450 - 512 nm.

The light emitted by the adjustable light source may thereby have a stimulating effect as the sensitivity of the melanopsin receptors is reported to be in the span of 450 - 520 nm, typically having a peak sensitivity in the range 470 - 490 nm. The light emitted by the adjustable light source may thereby increases cortisol and suppress melatonin in humans whereby an increasing attentiveness and focus may be achieved for a user illuminated by the illumination apparatus.

The adjustable light source may be a cold white light emitter and the second peak wavelength is within the range of 440 - 450 nm or 490 - 500 nm.

These peak wavelength ranges are near but outside the wavelength range of the first peak wavelength, i.e. 450- 490 nm. A lower suppression of melatonin may thereby be achieved by the light having the second peak wavelength. The light emitted by the adjustable light source may thereby have a less stimulating effect.

The database may thereby provide light recipes, i.e. directives, for achieving a given health condition and/or a behavior of an individual user having a given genomic data. The database may therefore be used to set a suitable light exposure for a new user provided that the genomic data of the new user matches or at least partly matches genomic data of the database. An improved starting point for achieving a desirable health condition and/or behavior of an individual user is further provided by the database providing information pertaining to the light exposure to be used to induce the desirable health condition and/or behavior.

To this end, it should be noted that the first database 109a and the second database 109b may be the same database.

The central control engine may further be configured to determine a point in time at which the individual user is exposed to the specific light profile.

The central control engine may further be configured to determine a time of exposure during which the individual user is exposed to the specific light profile.

The determining of the data set linking the specific light profile, genomic data pertaining to the individual user, and the data pertaining to the health condition and/or the behavior of the individual user sensed while exposing the individual user to the specific light profile may further comprise determining a point in time at which the individual user is exposed to the specific light profile.

An advantage being that the time the exposure took place may be determined. The point in time may further be correlated to the light exposure and stored in the database.

The point in time may further be understood as the time of day, day of week, and/or the day of a year.

By determining the time point and/or the duration of the light exposer a more accurate data base may be built up.

To this end, the determining of the data set linking the specific light profile, genomic data pertaining to the individual user, and the data pertaining to the health condition and/or the behavior of the individual user sensed while exposing the individual user to the specific light profile may further comprise determining a time of exposure during which the individual user is exposed to the specific light profile.

An advantage being that the dosage of the exposure may be determined. The time of exposure may further be stored in the database.

The linking each different specific light profile, genomic data pertaining to the specific individual user, and the data pertaining to the health condition and/or the behavior of the specific individual user sensed while exposing the specific individual user for each different specific light profile may further comprise points in time at which the individual user is respectively exposed to each different specific light profile.

The linking each different specific light profile, genomic data pertaining to the specific individual user, and the data pertaining to the health condition and/or the behavior of the specific individual user sensed while exposing the specific individual user for each different specific light profile may further comprise times of exposure during which the individual user is respectively exposed to each different specific light profile

A specific light profile may be selected from a set of predetermined specific light profiles. Light profiles known to influence the health condition and/or behavior of an individual may thereby be used.

Known light profiles may thereby be used for building up the database.

By way of example, genomic data comprising information on the PER3 gene polymorphisms may be used by the method and system described above. PER3 gene polymorphisms have been associated with differences in human sleep-wake phenotypes and sensitivity to light, see for example "Diurnal preference, mood and the response to morning light in relation to polymorphisms in the human clock gene PER3", Nature Sci Rep. 2017 Jul 31;7(1):6967. In short, the Period (PER) gene family is an important component of the biological clock. The human PER3 gene shows a high level of polymorphism suggesting that PER3 may account for individual differences in human circadian and sleep phenotypes. There is further evidence that, in humans, PER3 may be related to diurnal preference, or the personal inclination to be more/less active at different times of the waking day.

The method and system therefore offers the possibility to link genomic data comprising information on the PER gene polymorphisms with a specific light profile and data pertaining to the health condition and/or the behavior of the individual user.

The act of sensing may comprise one or more of: pulse, heart rate variability, blood pressure, sleep pattern, eating habits, facial expression, oxygen level, body posture, tone of voice, hormone levels, movement pattern, and productivity.

To achieve this the second sensor may be configured to sense pulse, heart rate variability, blood pressure, sleep pattern, eating habits, facial expression, oxygen level, body posture, tone of voice, hormone levels, movement pattern, and/or productivity.

The second sensor may be assigned to the specific user. With reference to "A practical guide to measuring physical activity" by L. G. Sylvia et al, J. Acad. Nutr Diet., 2014, 114(2), 199-208 different sensors exist for measuring the health status and/or behavior of a human.

To this end, the second sensor may be a thermometer. The temperature of the human or animal may be measured by the thermometer. The second sensor may be a thermistor.

The second sensor may be an accelerometer. The accelerometer may measure acceleration in real time and detect movement in up to three orthogonal planes. The accelerometer may be worn in numerous places on the body of the human or animal, including waist, hip, and thigh.

The second sensor may be a pedometer. The pedometers measure number of steps taken by the human or animal when the hip of the human or animal accelerates vertically with a force beyond a chosen threshold, typically measured with by the deflection of a horizontal, spring-suspended lever arm.

The second sensor may be a heart rate monitor. The heart rate monitor provides a physiological indicator of activity and energy expenditure. The heart rate monitor may provide real-time data on the frequency, duration, and/or intensity of activity of the human or animal.

The second sensor may be an armband. The armband may use motion and/or heat-related sensors, i.e., heat flux, galvanic skin response, skin temperature, body temperature, to measure energy expenditure and monitor activity of the human or animal.

The first sensor and/or the second sensor may comprise one or several light sensors. The light sensors may be arranged on the individual person to be worn or to be mounted at certain points in an area. The light sensors may be arranged to measure the intensity of light it is being exposed to and/or the spectral content of the light.

The central control engine may comprise a controller. The controller may be implemented using instructions that enable hardware functionality, for example, by using executable computer program instructions in a general-purpose or special-purpose processor that may be stored on a computer readable storage medium such as a memory to be executed by such a processor. The controller may be configured to read instructions from the memory and execute these instructions to control the operation of the light control system. The memory may be implemented using any commonly known technology for computer-readable memories such as ROM, RAM, SRAM, DRAM, CMOS, FLASH, DDR, SDRAM or some other memory technology.

The person skilled in the art realizes that the present invention by no means is limited to the preferred embodiments described above. On the contrary, many modifications and variations are possible within the scope of the appended claims.

For example, the individual user is above described to be a human, but may alternatively be an animal.

The term individual user should be interpreted broadly and may be understood as the individual user being a plant.

The wording "health condition" may therefore be understood as to relate to the pathology of the plant, i.e. the presence of a plant disease in the plant caused by pathogens, i.e., by infectious organisms, and/or environmental conditions, e.g. physiological factors such as light exposure, draught, or temperature.

The wording "behavior of an individual user". i.e. the behavior of the plant, may be understood as the response of to internal and external stimuli. The wording "behavior of an individual user" may therefore be understood as the physical state and/or motion associated with the plant. The physical state may, for example, relate to the form of the leaves of the plant. As an example curled or droopy leaves may imply that the plant is stressed. Yellow leaves may indicate that the plant has not produced enough chlorophyll. To this end, a plant with shiny, upright, and colorful leaves, e.g. dark green leaves may indicate that the plant is in a healthy state.

The observable motion may, for example, be the bending of a plant, e.g. the bending of the stem of the plant. As an example, plants such as seedlings which have stretched skinny stems and look fragile may lack sufficient light exposure by natural and/or artificial light. As a result, the plant may be bending forward rather than growing up straight with a strong stem.

The first sensor may be configured to identify an ID specific to the individual. The first sensor may be configured to read a QR, pin code, bar code, by which the individual user may be identified.

Additionally, variations to the disclosed embodiments can be understood and effected by the skilled person in practicing the claimed invention, from a study of the drawings, the disclosure, and the appended claims.

## Claims

1. A method for generating a database to be used to set illumination of an adjustable light source, the method (300) comprising:
for a plurality of different users:
identifying (302), by a first sensor (104), an individual user (110);
setting (304) a specific light profile of the adjustable light source (102), the specific light profile comprising information pertaining to a spectral content and/or intensity of light by which the individual user (110) is exposed (112) to;
while exposing (112) the individual user (110) to the specific light profile, sensing (306), by a second sensor (106), data pertaining to a health condition and/or a behavior of the individual user (110), wherein the act of sensing (306) comprises sensing one or more of: pulse, heart rate variability, blood pressure, sleep pattern, eating habits, facial expression, oxygen level, body posture, tone of voice, hormone levels, movement pattern, and productivity;
the method is **characterized in that** for the plurality of different users the method further comprises:
determining (308) a data set linking the specific light profile, genomic data pertaining to the individual user (110), and the data pertaining to the health condition and/or the behavior of the individual user sensed while exposing (112) the individual user (110) to the specific light profile; and
storing (310) the linked data set in the database.

2. The method according to claim 1, wherein the determining (308) of the data set linking the specific light profile, genomic data pertaining to the individual user (110), and the data pertaining to the health condition and/or the behavior of the individual user sensed while exposing (112) the individual user (110) to the specific light profile further comprises determining a point in time at which the individual user (110) is exposed to the specific light profile.

3. The method according to claim 1, wherein the determining (308) of the data set linking the specific light profile, genomic data pertaining to the individual user (110), and the data pertaining to the health condition and/or the behavior of the individual user (110) sensed while exposing the individual user (110) to the specific light profile further comprises determining a time of exposure during which the individual user is exposed to the specific light profile.

4. The method according to any one of claims 1 - 3, further comprising, for a specific individual user:
exposing (312) the specific individual user (110) for different specific light profiles (406a, 406b);
sensing data (314) pertaining to a health condition and/or a behavior of the specific individual user for each different specific light profile; and
linking (316) each different specific light profile (406a, 406b), genomic data pertaining to the specific individual user (110), and the data pertaining to the health condition and/or the behavior of the specific individual user (110) sensed while exposing (112, 408) the specific individual user (110) for each different specific light profile (406a, 406b).

5. The method according to claim 3, wherein the linking (316) each different specific light profile (406a, 406b), genomic data pertaining to the specific individual user (110), and the data pertaining to the health condition and/or the behavior of the specific individual user (110) sensed while exposing the specific individual (110) user for each different specific light profile further comprises points in time at which the individual user (110) is respectively exposed to each different specific light profile.

6. The method according to claim 3, wherein the linking (316) each different specific light profile (406a, 406b), genomic data pertaining to the specific individual user (110), and the data pertaining to the health condition and/or the behavior of the specific individual user (110) sensed while exposing the specific individual user (110) for each different specific light profile (406a, 406b) further comprises times of exposure during which the individual user (110) is respectively exposed to each different specific light profile.

7. The method according to any one of claims 1 - 6, wherein a specific light profile is selected from a set of predetermined specific light profiles.

8. A light control system comprising:
an adjustable light source (102), wherein the light source (102) is adjustable in spectral content and/or intensity of light emitted;
a first sensor (104) configured to identify (302) an individual user (110) among a plurality of individual users;
a central control engine (108) configured to set (304) a specific light profile of the adjustable light source (102), the specific light profile comprising information pertaining to a spectral content and/or intensity of light by which the individual user (110) is exposed to; and
a second sensor (106) configured to sense data (306) pertaining to a health condition and/or a behavior of the individual user (110) while exposing the individual user (110) to the specific light profile, wherein the second sensor (106) is configured to sense one or more of pulse, heart rate variability, blood pressure, sleep pattern, eating habits, facial expression, oxygen level, body posture, tone of voice, hormone levels, movement pattern, and productivity;
the light control system being **characterized in:**
**that** it further comprises a first database (109a) comprising genomic data pertaining to each of the plurality of individual users; and
**that** the central control engine (108) is further configured to:
determine (308) a data set linking the specific light profile, genomic data pertaining to the individual user (110), and the data pertaining to the health condition and/or the behavior of the individual user (110) sensed by the second sensor (106); and
store (310) the linked data set in a second database.

9. The light control system of claim 8, wherein the first sensor (104) and second sensor (106) is the same sensor.

10. The light control system of claim 8, wherein the first sensor (104) is a camera.

11. The light control system of claim 8, wherein the central control engine (108) is further configured to determine a point in time at which the individual user (110) is exposed (112) to the specific light profile.

12. The light control system of claim 8, wherein the central control engine (108) is further configured to determine a time of exposure during which the individual user (110) is exposed to the specific light profile.

13. The light control system of claim 8, wherein the first database (109a) and the second database (109b) is the same database.

## Patentansprüche

1. Verfahren zum Erzeugen einer Datenbank, die dazu bestimmt ist, zum Einstellen der Beleuchtung einer regulierbaren Lichtquelle verwendet zu werden, wobei das Verfahren (300) Folgendes umfasst:
für eine Vielzahl verschiedener Benutzer:
Identifizieren (302), durch einen ersten Sensor (104), eines einzelnen Benutzers (110);
Einstellen (304) eines spezifischen Lichtprofils der regulierbaren Lichtquelle (102), wobei das spezifische Lichtprofil Informationen betreffend einen Spektralgehalt und/oder eine Lichtintensität umfasst, denen der einzelne Benutzer (110) ausgesetzt wird (112);
während der einzelne Benutzer (110) dem spezifischen Lichtprofil ausgesetzt wird (112), Erfassen (306), durch einen zweiten Sensor (106), von Daten betreffend einen Gesundheitszustand und/oder ein Verhalten des einzelnen Benutzers (110), wobei der Vorgang des Erfassens (306) Erfassen eines oder mehrerer der Folgenden umfasst: Puls, Variabilität des Herzschlags, Blutdruck, Schlafmuster, Essverhalten, Gesichtsausdruck, Sauerstoffgehalt, Körperhaltung, Tonalität, Hormonlage, Bewegungsmuster und Produktivität;
wobei das Verfahren **dadurch gekennzeichnet ist, dass** das Verfahren für die Vielzahl verschiedener Benutzer ferner Folgendes umfasst:
Bestimmen (308) eines Datensatzes, der das spezifische Lichtprofil, genomische Daten betreffend den einzelnen Benutzer (110) und die Daten betreffend den Gesundheitszustand und/oder das Verhalten des einzelnen Benutzers zusammenführt, die erfasst wurden, während der einzelne Benutzer (110) dem spezifischen Lichtprofil ausgesetzt wurde (112); und
Speichern (310) des zusammengeführten Datensatzes in der Datenbank.

2. Verfahren nach Anspruch 1, wobei das Bestimmen (308) des Datensatzes, der das spezifische Lichtprofil, genomische Daten betreffend den einzelnen Benutzer (110) und die Daten betreffend den Gesundheitszustand und/oder das Verhalten des einzelnen Benutzers zusammenführt, die erfasst wurden, während der einzelne Benutzer (110) dem spezifischen Lichtprofil ausgesetzt wurde (112), ferner Bestimmen eines Zeitpunkts umfasst, zu dem der einzelne Benutzer (110) dem spezifischen Lichtprofil ausgesetzt wird.

3. Verfahren nach Anspruch 1, wobei das Bestimmen (308) des Datensatzes, der das spezifische Lichtprofil, genomische Daten betreffend den einzelnen Benutzer (110) und die Daten betreffend den Gesundheitszustand und/oder das Verhalten des einzelnen Benutzers zusammenführt, die erfasst wurden, während der einzelne Benutzer (110) dem spezifischen Lichtprofil ausgesetzt wurde (112), ferner Bestimmen einer Expositionsdauer umfasst, während der der einzelne Benutzer (110) dem spezifischen Lichtprofil ausgesetzt wird.

4. Verfahren nach einem der Ansprüche 1-3, ferner umfassend für einen spezifischen einzelnen Benutzer:
Aussetzen (312) des spezifischen einzelnen Benutzers (110) gegenüber verschiedenen spezifischen Lichtprofilen (406a, 406b);
Erfassen von Daten (314) betreffend einen Gesundheitszustand und/oder ein Verhalten des spezifischen einzelnen Benutzers für jedes der verschiedenen spezifischen Lichtprofile; und
Zusammenführen (316) jedes der verschiedenen spezifischen Lichtprofile (406a, 406b) mit genomischen Daten betreffend den spezifischen einzelnen Benutzer (110) und den Daten betreffend den Gesundheitszustand und/oder das Verhalten des spezifischen einzelnen Benutzers (110), die erfasst wurden, während der spezifische einzelne Benutzer (110) jedem der verschiedenen spezifischen Lichtprofile (406a, 406b) ausgesetzt wurde (112, 408).

5. Verfahren nach Anspruch 3, wobei das Zusammenführen (316) jedes der verschiedenen spezifischen Lichtprofile (406a, 406b) mit genomischen Daten betreffend den spezifischen einzelnen Benutzer (110) und den Daten betreffend den Gesundheitszustand und/oder das Verhalten des spezifischen einzelnen Benutzers (110), die erfasst wurden, während der spezifische einzelne Benutzer (110) jedem der verschiedenen spezifischen Lichtprofile ausgesetzt wurde, ferner Zeitpunkte umfasst, zu denen der einzelne Benutzer (110) jeweils jedem der verschiedenen spezifischen Lichtprofile ausgesetzt wird.

6. Verfahren nach Anspruch 3, wobei das Zusammenführen (316) jedes der verschiedenen spezifischen Lichtprofile (406a, 406b) mit genomischen Daten betreffend den spezifischen einzelnen Benutzer (110) und den Daten betreffend den Gesundheitszustand und/oder das Verhalten des spezifischen einzelnen Benutzers (110), die erfasst wurden, während der spezifische einzelne Benutzer (110) jedem der verschiedenen spezifischen Lichtprofile (406a, 406b) ausgesetzt wurde, ferner Expositionsdauern umfasst, während denen der einzelne Benutzer (110) jeweils jedem der verschiedenen spezifischen Lichtprofile ausgesetzt wird.

7. Verfahren nach einem der Ansprüche 1-6, wobei ein spezifisches Lichtprofil aus einem Satz vorbestimmter spezifischer Lichtprofile ausgewählt wird.

8. Lichtsteuerungssystem, umfassend:
eine regulierbare Lichtquelle (102), wobei die Lichtquelle (102) bezüglich des Spektralgehalts und/oder der Intensität des abgegebenen Lichtes regulierbar ist;
einen ersten Sensor (104), der dazu konfiguriert ist, einen einzelnen Benutzer (110) unter einer Vielzahl einzelner Benutzer zu identifizieren (302);
ein zentrales Steuergerät (108), das dazu konfiguriert ist, ein spezifisches Lichtprofil der regulierbaren Lichtquelle (102) einzustellen (304), wobei das spezifische Lichtprofil Informationen betreffend einen Spektralgehalt und/oder eine Lichtintensität umfasst, denen der einzelne Benutzer (110) ausgesetzt wird; und
einen zweiten Sensor (106), der dazu konfiguriert ist, Daten (306) betreffend einen Gesundheitszustand und/oder ein Verhalten des einzelnen Benutzers (110) zu erfassen, während der einzelne Benutzer (110) dem spezifischen Lichtprofil ausgesetzt wird, wobei der zweite Sensor (106) dazu konfiguriert ist, eines oder mehrere der Folgenden zu erfassen: Puls, Variabilität des Herzschlags, Blutdruck, Schlafmuster, Essverhalten, Gesichtsausdruck, Sauerstoffgehalt, Körperhaltung, Tonalität, Hormonlage, Bewegungsmuster und Produktivität;
wobei das Lichtsteuerungssystem **dadurch gekennzeichnet ist,**
**dass** es ferner eine erste Datenbank (109a) umfasst, die genomische Daten umfasst, die jeden der Vielzahl einzelner Benutzer betreffen; und
**dass** das zentrale Steuergerät (108) ferner dazu konfiguriert ist:
einen Datensatz zu bestimmen (308), der das spezifische Lichtprofil, genomische Daten betreffend den einzelnen Benutzer (110) und die Daten betreffend den Gesundheitszustand und/oder das Verhalten des einzelnen Benutzers (110) zusammenführt, die von dem zweiten Sensor (106) erfasst wurden; und
den zusammengeführten Datensatz in einer zweiten Datenbank zu speichern (310).

9. Lichtsteuerungssystem nach Anspruch 8, wobei der erste Sensor (104) und der zweite Sensor (106) der gleiche Sensor sind.

10. Lichtsteuerungssystem nach Anspruch 8, wobei der erste Sensor (104) eine Kamera ist.

11. Lichtsteuerungssystem nach Anspruch 8, wobei das zentrale Steuergerät (108) ferner dazu konfiguriert ist, einen Zeitpunkt zu bestimmen, zu dem der einzelne Benutzer (110) dem spezifischen Lichtprofil ausgesetzt wird (112).

12. Lichtsteuerungssystem nach Anspruch 8, wobei das zentrale Steuergerät (108) ferner dazu konfiguriert ist, eine Expositionsdauer zu bestimmen, während der der einzelne Benutzer (110) dem spezifischen Lichtprofil ausgesetzt wird.

13. Lichtsteuerungssystem nach Anspruch 8, wobei die erste Datenbank (109a) und die zweite Datenbank (109b) dieselbe Datenbank sind.

## Revendications

1. Procédé de génération d'une base de données à utiliser pour régler l'éclairage d'une source de lumière réglable, le procédé (300) comprenant :
pour une pluralité d'utilisateurs différents :
l'identification (302), par un premier capteur (104), d'un utilisateur individuel (110) ;
la définition (304) d'un profil de lumière spécifique de la source de lumière réglable (102), le profil de lumière spécifique comprenant des informations relatives à un contenu spectral et/ou à une intensité de lumière à laquelle l'utilisateur individuel (110) est exposé (112) ;
tout en exposant (112) l'utilisateur individuel (110) au profil de lumière spécifique, la détection (306), par un second capteur (106), de données relatives à un état de santé et/ou à un comportement de l'utilisateur individuel (110), dans lequel l'acte de détection (306) comprend la détection d'un ou de plusieurs éléments parmi : le pouls, la variabilité de la fréquence cardiaque, la tension artérielle, le rythme de sommeil, les habitudes alimentaires, l'expression du visage, le niveau d'oxygène, la posture du corps, le ton de la voix, les niveaux d'hormones, le schéma de mouvement et la productivité ;
le procédé est **caractérisé en ce que** pour la pluralité d'utilisateurs différents, le procédé comprend en outre :
la détermination (308) d'un ensemble de données liant le profil de lumière spécifique, des données génomiques relatives à l'utilisateur individuel (110) et des données relatives à l'état de santé et/ou au comportement de l'utilisateur individuel détectées lors de l'exposition (112) de l'utilisateur individuel (110) au profil de lumière spécifique ; et
le stockage (310) de l'ensemble de données liées dans la base de données.

2. Procédé selon la revendication 1, dans lequel la détermination (308) de l'ensemble de données liant le profil de lumière spécifique, des données génomiques relatives à l'utilisateur individuel (110) et des données relatives à l'état de santé et/ou au comportement de l'utilisateur individuel détectées lors de l'exposition (112) de l'utilisateur individuel (110) au profil de lumière spécifique comprend en outre la détermination d'un moment auquel l'utilisateur individuel (110) est exposé au profil de lumière spécifique.

3. Procédé selon la revendication 1, dans lequel la détermination (308) de l'ensemble de données liant le profil de lumière spécifique, des données génomiques relatives à l'utilisateur individuel (110) et des données relatives à l'état de santé et/ou au comportement de l'utilisateur individuel (110) détectées lors de l'exposition de l'utilisateur individuel (110) au profil de lumière spécifique comprend en outre la détermination d'un moment d'exposition pendant lequel l'utilisateur individuel est exposé au profil de lumière spécifique.

4. Procédé selon l'une quelconque des revendications 1 à 3, comprenant en outre, pour un utilisateur individuel spécifique :
l'exposition (312) de l'utilisateur individuel spécifique (110) à différents profils de lumière spécifiques (406a, 406b) ;
la détection de données (314) relatives à un état de santé et/ou à un comportement de l'utilisateur individuel spécifique pour chaque profil de lumière spécifique différent ; et
la liaison (316) de chaque profil de lumière spécifique différent (406a, 406b), des données génomiques relatives à l'utilisateur individuel (110) spécifique et des données relatives à l'état de santé et/ou au comportement de l'utilisateur individuel (110) spécifique détectées lors de l'exposition (112, 408) de l'utilisateur individuel (110) spécifique pour chaque profil de lumière spécifique différent (406a, 406b).

5. Procédé selon la revendication 3, dans lequel la liaison (316) de chaque profil de lumière spécifique différent (406a, 406b), de données génomiques relatives à l'utilisateur individuel (110) spécifique, et des données relatives à l'état de santé et/ou au comportement de l'utilisateur individuel (110) spécifique détectées lors de l'exposition de l'utilisateur individuel (110) spécifique pour chaque profil de lumière spécifique différent comprend en outre des moments auxquels l'utilisateur individuel (110) est respectivement exposé à chaque profil de lumière spécifique différent.

6. Procédé selon la revendication 3, dans lequel la liaison (316) de chaque profil de lumière spécifique différent (406a, 406b), de données génomiques relatives à l'utilisateur individuel (110) spécifique, et des données relatives à l'état de santé et/ou au comportement de l'utilisateur individuel (110) spécifique détectées lors de l'exposition de l'utilisateur individuel (110) spécifique pour chaque profil de lumière spécifique différent (406a, 406b) comprend en outre des moments d'exposition pendant lesquels l'utilisateur individuel (110) est respectivement exposé à chaque profil de lumière spécifique différent.

7. Procédé selon l'une quelconque des revendications 1 à 6, dans lequel un profil de lumière spécifique est sélectionné parmi un ensemble de profils de lumière spécifiques prédéterminés.

8. Système de commande d'éclairage comprenant :
une source de lumière réglable (102), dans lequel la source de lumière (102) est réglable en termes de contenu spectral et/ou d'intensité de lumière émise ;
un premier capteur (104) configuré pour identifier (302) un utilisateur individuel (110) parmi une pluralité d'utilisateurs individuels ;
un moteur de commande central (108) configuré pour définir (304) un profil de lumière spécifique de la source de lumière réglable (102), le profil de lumière spécifique comprenant des informations relatives à un contenu spectral et/ou à une intensité de lumière à laquelle l'utilisateur individuel (110) est exposé ; et
un second capteur (106) configuré pour détecter des données (306) relatives à un état de santé et/ou à un comportement de l'utilisateur individuel (110) tout en exposant l'utilisateur individuel (110) au profil de lumière spécifique, dans lequel le second capteur (106) est configuré pour détecter un ou plusieurs éléments parmi le pouls, la variabilité de la fréquence cardiaque, la pression artérielle, le rythme de sommeil, les habitudes alimentaires, l'expression faciale, le niveau d'oxygène, la posture du corps, le ton de la voix, les niveaux d'hormones, le schéma de mouvement et la productivité ;
le système de commande d'éclairage étant **caractérisé :**
**en ce qu'**il comprend en outre une première base de données (109a) comprenant des données génomiques appartenant à chacun de la pluralité d'utilisateurs individuels ; et
**en ce que** le moteur de commande central (108) est en outre configuré pour :
déterminer (308) un ensemble de données liant le profil de lumière spécifique, des données génomiques relatives à l'utilisateur individuel (110), et des données relatives à l'état de santé et/ou au comportement de l'utilisateur individuel (110) détectées par le second capteur (106) ; et
stocker (310) l'ensemble de données liées dans une seconde base de données.

9. Système de commande de lumière selon la revendication 8, dans lequel le premier capteur (104) et le second capteur (106) sont le même capteur.

10. Système de commande de lumière selon la revendication 8, dans lequel le premier capteur (104) est une caméra.

11. Système de commande de lumière selon la revendication 8, dans lequel le moteur de commande central (108) est en outre configuré pour déterminer un moment auquel l'utilisateur individuel (110) est exposé (112) au profil de lumière spécifique.

12. Système de commande de lumière selon la revendication 8, dans lequel le moteur de commande central (108) est en outre configuré pour déterminer un moment d'exposition pendant lequel l'utilisateur individuel (110) est exposé au profil de lumière spécifique.

13. Système de commande de lumière selon la revendication 8, dans lequel la première base de données (109a) et la seconde base de données (109b) sont la même base de données.
